# EUROPEAN PATENT SPECIFICATION

(11) **EP 2 286 826 B1**
(45) Date of publication and mention of the grant of the patent: **04.01.2017**
(21) Application number: 10009648.6
(22) Date of filing: 29.10.2001
(51) Int. Cl.: A61K 38/20, A61P 35/00, A61K 31/541, A61K 31/549

(54) **Treatment of tumor metastases and cancer**
Behandlung von Tumor-Metastasen und Krebs
Traitement de métastases tumorales et de cancers

(30) Priority: 27.10.2000 US 243409 P
(43) Date of publication of application: 23.02.2011
(62) Divisional of application: 09009373.3
(73) Proprietor: ED. GEISTLICH SÖHNE AG FÜR CHEMISCHE INDUSTRIE, 6110 Wolhusen (CH)
(72) Inventor: Redmond, Paul, H., Wilton, Cork (IE); Pfirrmann, Rolf, W., 6353 Weggis (CH)
(74) Representative: Matthews, Derek Peter

(56) References cited:
- EP-A- 1 066 830
- WO-A-92/00743
- WO-A2-01/39763

## Description

The present invention relates to the field of treating tumor metastases and cancer.

Interleukin-2 (IL-2) is an agent which has been suggested for inhibiting tumor cell growth. However, administration of IL-2 to patients presents severe toxicity problems, since IL-2 elicits an extremely strong systemic inflammatory response syndrome (SIRS) reaction in patients. Toxicity of IL-2 is so severe that approximately 70% of patients cannot tolerate treatment.

Additionally, a common problem in patients undergoing cancer treatment is tumor recurrence or metastasis.

Thus, despite the advances in cancer treatment, there remains a significant need in the art for new and improved cancer treatment therapies.

In accordance with the present invention, tumor metastasis is inhibited in a cancer patient by administering to said patient a combination therapy comprising effective amounts of IL-2 and a methylol transfer agent selected from taurolidine and/or taurultam.

It has surprisingly been found that taurolidine and taurultam can reduce or substantially eliminate the severe toxicity and side effects of IL-2 in a combination therapy for inhibiting tumor metastases and treating cancer in patients, while it has unexpectedly been found that the efficacy of IL-2 is actually enhanced by the methylol transfer agents in the combination therapy of the present invention.

IL-2 when used in accordance with the present invention includes natural or recombinant Interleukin-2, or biologically active derivatives or substantial equivalents thereof.

Taurolidine and taurultam have been disclosed as anticancer agents in WO92/00743 and EP-A-1066830. Other anticancer methylol-containing compounds may be found among those identified in WO 01/39763.

The invention therefore provides the use of (i) taurolidine and/or taurultam and (ii) interleukin-2 in the manufacture of a combined preparation for simultaneously, separately or sequentially treating breast cancer or pancreatic cancer.

Effective daily dosage amounts of IL-2 may comprise pharmaceutical dosage units within the range of 1,000,000-100,000,000 units (U) IL-2 per m² body surface area. Dosage amounts of IL-2 also may be found within the range of 100,000-1,000,000 U per kilogram body weight. Dosage amounts of IL-2 further may be found within the range of 0.1-100 micrograms IL-2 per kilogram body weight.

Effective dosage amounts of the methylol transfer agent in accordance with the present invention may comprise pharmaceutical dosage units within the range of about 0.1-1,000 mg/kg. Preferred dosages may be in the range of about 10-20 grams per administration.

Pharmaceutical dosage units of the combined therapy of the present invention may be administered by any suitable route, which include oral, topical or peritoneal administration, e.g., subcutaneously, intraperitoneally, intramuscularly, or intravenously, e.g. by infusion or injection.

In preferred embodiments, 250 ml of taurolidine 2% solution is administered by intravenous infusion about 1-6 times per day, more preferably about 2-4 times per day, during a treatment period, concurrently with administration of about 10,000,000-40,000,000 units/m² IL-2 by intravenous infusion per day during the treatment period.

The present invention is thus directed to a combination of IL-2 and the methylol transfer agent, in effective amounts for simultaneous, separate or sequential use for inhibiting tumor metastasis in a cancer patient. The invention is also directed to pharmaceutical combinations including pharmaceutical dosage units comprising effective amounts of Interleukin-2 and the methylol transfer agent for inhibiting tumor metastasis in a cancer patient, as well as to pharmaceutical compositions comprising such combinations. According to the invention, the patient is suffering from breast cancer or pancreatic cancer.

The following examples are for reference only.

### Example 1

A 63 year old patient diagnosed with metastatic malignant melanoma was treated as follows.
- Presentation: Right supra-clavicular mass. Originally had nodular melanoma excised from right elbow, and had high-dose interferon post-operatively. Required axillary clearance for a mass in right axilla eight months later. Further staging was clear at that time. Presented one year later with a fixed inoperable mass in right supra-clavicular area.
- Treatment: IL-2 and Taurolidine
- Regimen: Interleukin-2
Day 1: 18 million units/m² IL-2 infusion over 6 hours
Day 2: 18 million units/m² IL-2 infusion over 12 hours
Day 3: 18 million units/m² IL-2 infusion over 24 hours
Days 4-7: 18 million units/m² IL-2 infusion over 78 hours
Taurolidine
Taurolidine 2% 250 ml infusion over twelve hours, daily during IL-2 administration
Completed five courses of the above

After one year, the patient is alive and well, with no evidence of disease on imaging.

### Example 2

A 50 year old patient diagnosed with metastatic renal cell carcinoma was treated as follows.
- Presentation: Haemoptysis - 2° to pulmonary metastases. Noted to have hepatic metastases, in addition to a large mass in the left kidney.
- Treatment: IL-2 and Taurolidine
- Regimen: Interleukin-2
Day 1: 18 million units/m² IL-2 infusion over 6 hours
Day 2: 18 million units/m² IL-2 infusion over 12 hours
Day 3: 18 million units/m² IL-2 infusion over 24 hours
Days 4-7: 18 million units/m² IL-2 infusion over 78 hours
Taurolidine
Taurolidine 2% 250 ml infusion over two hours, twice daily during IL-2 administration
Completed five courses of the above

### Further treatment Left radical nephrectomy

After five years, the patient is alive and well, with no evidence of disease on imaging.

### Example 3

A male patient who had recurrent nodular melanoma after interferon treatment was subsequently treated with Interleukin-2 and Taurolidine as follows:
- Presentation: Recurrence of nodular melanoma lesion in right shoulder.
- Treatment: IL-2 and Taurolidine
- Regimen: Interleukin-2
Day 1: 36 million units/m² IL-2 infusion over 6 hours
Day 2: 36 million units/m² IL-2 infusion over 12 hours
Day 3: 36 million units/m² IL-2 infusion over 24 hours
Days 4-7: 36 million units/m² IL-2 infusion over 78 hours
Taurolidine
Taurolidine 2% 250 ml infusion over twelve hours, sequentially with IL-2 administration, during days 1-6
Undertook five courses -- during second course, treatment was
interrupted and stopped at 78 hours, and during the fifth course, treatment was interrupted during day 4.

Follow-up CT scans indicated a reduction in the size of the lesion, and subsequently indicated no evidence of disease.

## Claims

1. Use of (i) taurolidine and/or taurultam and (ii) interleukin-2 in the manufacture of a combined preparation for simultaneously, separately or sequentially treating breast cancer or pancreatic cancer.

2. The use of claim 1 wherein said combined preparation is formulated for simultaneous administration of taurolidine and/or taurultam, and interleukin-2.

3. The use of claim 1 wherein said combined preparation is formulated for sequential administration of taurolidine and/or taurultam, and interleukin-2.

4. The use of claim 1 wherein said combined preparation is formulated for separate administration of taurolidine and/or taurultam, and interleukin-2.

5. The use of claim 1 wherein said combined preparation contains dosage units which provide effective daily dosage amounts of 1,000,000 - 100,000,000 units interleukin-2 per m² body surface area.

6. The use of claim 1 wherein said combined preparation provides dosage amounts of 0.1-100 micrograms interleukin-2 per kilogram body weight.

7. The use of claim 1 wherein said combined preparation contains dosage units which provide effective dosage amounts of 0.1-1000 mg/kg taurolidine and/or taurultam.

8. The use of claim 1 wherein said combined preparation provides 10-20 g taurolidine and/or taurultam per administration.

9. The use of claim 1 wherein said combined preparation is formulated for administration orally, topically, intraperitoneally, subcutaneously, intramuscularly, intravenously, or by infusion.

## Patentansprüche

1. Verwendung von (i) Taurolidin und/oder Taurultam und (ii) Interleukin-2 bei der Herstellung eines Kombinationspräparats zur simultanen, getrennten oder sequenziellen Behandlung von Brustkrebs oder Baumspeicheldrüsenkrebs.

2. Verwendung nach Anspruch 1, wobei das Kombinationspräparat zur simultanen Verabreichung von Taurolidin und/oder Taurultam und Interleukin-2 formuliert wird.

3. Verwendung nach Anspruch 1, wobei das Kombinationspräparat zur simultanen Verabreichung von Taurolidin und/oder Taurultam und Interleukin-2 formuliert wird.

4. Verwendung nach Anspruch 1, wobei das Kombinationspräparat zur getrennten Verabreichung von Taurolidin und/oder Taurultam und Interleukin-2 formuliert.

5. Verwendung nach Anspruch 1, wobei das Kombinationspräparat Dosierungseinheiten enthält, die wirksame tägliche Dosierungsmengen von 1,000,000- 100,000,000 Einheiten Interleukin-2 pro m² Körperoberflächenbereich bereitstellen.

6. Verwendung nach Anspruch 1, wobei das Kombinationspräparat Dosierungsmengen von 0,1 - 100 Mikrogramm Interleukin-2 pro Kilogramm Körpergewicht bereitstellt.

7. Verwendung nach Anspruch 1, wobei das Kombinationspräparat Dosierungseinheiten enthält, die wirksame Dosierungsmengen von 0,1 - 1,000 mg/kg Taurolidin und/oder Taurultam bereitstellen.

8. Verwendung nach Anspruch 1, wobei das Kombinationspräparat 10 - 20 g Taurolidin und/oder Taurultam pro Verabreichung bereitstellt.

9. Verwendung nach Anspruch 1, wobei das Kombinationspräparat zur Verabreichung oral, topisch, intraperitoneal, subkutan, intramuskulär, intravenös oder durch Infusion formuliert ist.

## Revendications

1. Utilisation de (i) taurolidine et/ou taurultam et (ii) d'interleukine 2 dans la fabrication d'une préparation combinée pour simultanément, séparément ou séquentiellement traiter un cancer du sein ou un cancer pancréatique.

2. Utilisation selon la revendication 1, dans laquelle ladite préparation combinée est formulée pour l'administration simultanée de taurolidine et/ou de taurultam et d'interleukine 2.

3. Utilisation selon la revendication 1, dans laquelle ladite préparation combinée est formulée pour l'administration séquentielle de taurolidine et/ou de taurultam et d'interleukine 2.

4. Utilisation selon la revendication 1, dans laquelle ladite préparation combinée est formulée pour l'administration séparée de taurolidine et/ou de taurultam et d'interleukine 2.

5. Utilisation selon la revendication 1, dans laquelle ladite préparation combinée contient des unités de dosage qui fournissent des quantités de dosage quotidiennes efficaces de 1 000 000 à 100 000 000 unités d'interleukine 2 par m² de superficie corporelle.

6. Utilisation selon la revendication 1, dans laquelle ladite préparation combinée fournit des quantités de dosage de 0,1 à 100 microgrammes d'interleukine 2 par kilogramme de poids corporel.

7. Utilisation selon la revendication 1, dans laquelle ladite préparation combinée contient des unités de dosage qui fournissent des quantités de dosage efficaces de 0,1 à 1000 mg/kg de taurolidine et/ou taurultam.

8. Utilisation selon la revendication 1, dans laquelle ladite préparation combinée fournit 10 à 20 g de taurolidine et/ou taurultam par administration.

9. Utilisation selon la revendication 1, dans laquelle ladite préparation combinée est formulée pour une administration par voie orale, voie topique, intra-péritonéale, sous-cutanée, intramusculaire, intraveineuse, ou par perfusion.
